# EUROPEAN PATENT APPLICATION

(11) **EP 4 799 676 A1**
(43) Date of publication of application: **02.09.2026**
(21) Application number: 24881943.5
(22) Date of filing: 19.03.2024
(51) Int. Cl.: A61N 1/36, A61N 1/04

(54) **DEVICE AND ATTACHMENT FOR TRANSCUTANEOUS ELECTRICAL STIMULATION OF SECOND DIVISION AND/OR THIRD DIVISION OF TRIGEMINAL NERVE**

(30) Priority: 27.10.2023 JP 2023184896
(71) Applicant: Piculette Inc., Tokyo 104-0061 (JP)
(72) Inventor: MUTO, Manabu, Nagoya-shi, Aichi 464-8601 (JP); FUSE, Yutaro, Nagoya-shi, Aichi 464-8601 (JP); SAITO, Ryuta, Nagoya-shi, Aichi 464-8601 (JP); OYAMA, Shintaro, Nagoya-shi, Aichi 464-8601 (JP)
(74) Representative: Heyerhoff Geiger GmbH & Co. KG
(86) International application number: PCT/JP2024/010634
(87) International publication number: WO 2025/088825

(57) **Abstract**

Provided are a device and attachment for transcutaneous electrical stimulation of the second division and/or the third division of the trigeminal nerve, comprising electrodes held on a glasses-shaped frame (2) and an electric circuit for applying electric pulses to the electrode. The device and attachment comprise at least one electrode portion selected from a first electrode portion (31) including at least two electrodes for selectively electrically stimulating the right facial second division, a second electrode portion (32) including at least two electrodes for selectively electrically stimulating the left facial second division, a third electrode portion (33) including at least two electrodes for selectively electrically stimulating the right facial third division, and a fourth electrode portion (34) including at least two electrodes for selectively electrically stimulating the left facial third division. The electric circuit forms a circuit between the at least two electrodes of the first electrode portion (31), forms a circuit between the at least two electrodes of the second electrode portion (32), forms a circuit between the at least two electrodes of the third electrode portion (33), or forms a circuit between the at least two electrodes of the fourth electrode portion (34).

## Description

### [Technical Field]

The disclosure of the present application relates to a device for applying transcutaneous electrical nerve stimulation to a second branch and/or a third branch of a trigeminal nerve and relates to an attachment for applying transcutaneous electrical nerve stimulation to the second branch and/or the third branch of the trigeminal nerve.

### [Background Art]

Devices for applying transcutaneous electrical nerve stimulation to the trigeminal nerve are known. For example, Patent Literature 1 discloses a device that applies transcutaneous electrical nerve stimulation to the first branch of the trigeminal nerve. Further, Patent Literature 2 discloses a system for trigeminal nerve stimulation including a first electrode including at least one contact point configured for installation on the skin in a first region of the face of a patient covering a cutaneous distribution of at least one branch of the trigeminal nerve.

Further, Patent Literature 3 discloses an eyewear for detecting biological signals such as electrooculograms through this eyewear is not a device that applies transcutaneous electrical nerve stimulation to the trigeminal nerve.

### [Citation List]

### [Patent Literature]

Patent Literature 1: Japanese Patent No. 6606270
Patent Literature 2: Japanese Patent No. 6130789
Patent Literature 3: Japanese Patent No. 6306926

### [Summary of Invention]

### [Technical Problem]

The device disclosed in Patent Literature 1 listed above is a device that applies transcutaneous electrical nerve stimulation to the first branch of the trigeminal nerve and is used in such a way that a long symmetrical support having at least one electrode pair is attached to the forehead. Thus, there is a problem that, in use of the device disclosed in Patent Literature 1, the user is unable to externally hide that he/she is wearing a distinctly unusual device and it is difficult for the user to use the device without worrying about the appearance from others. Further, there is a problem that, since the device disclosed in Patent Literature 1 is a device that applies transcutaneous electrical nerve stimulation dedicatedly to the first branch, it is not possible to apply transcutaneous electrical nerve stimulation to the second branch and/or the third branch.

The device disclosed in Patent Literature 2 is also the device used by attaching an electrode assembly to the forehead as illustrated in FIG. 4 of Patent Literature 2 or attaching two separate electrodes to the forehead as illustrated in FIG. 7 of Patent Literature 2. Therefore, there is a problem as with Patent Literature 1 that it is difficult for the user to use the device without worrying about the appearance from others. Further, although the device disclosed in Patent Literature 2 is described as being used to cover a cutaneous distribution of at least one branch of the trigeminal nerve, only an embodiment in which the device is attached to the forehead as illustrated in FIG. 4 and FIG. 7 is disclosed in the specification. Patent Literature 2 does not at all disclose what embodiment is preferable in applying transcutaneous electrical nerve stimulation to the second branch and/or the third branch.

The device disclosed in Patent Literature 3 is an eyeglass-type device for detecting biological signals such as electrooculograms as described above. Thus, in the device disclosed in Patent Literature 3, there is a problem that it is not possible to apply transcutaneous electrical nerve stimulation to the trigeminal nerve.

The disclosure of the present application has been made to solve the above problems. The present inventors have made intensive studies and thus newly found that the above problems can be solved by a configuration in which at least a part of an electrode unit used for applying transcutaneous electrical nerve stimulation to the second branch and/or the third branch of the trigeminal nerve is held on an eyeglass-type frame.

That is, the object of the disclosure of the present application is to provide a device for applying transcutaneous electrical nerve stimulation to the second branch and/or the third branch of the trigeminal nerve and provide an attachment that allows commercially available eyeglasses to function as the device for applying transcutaneous electrical nerve stimulation to the second branch and/or the third branch of the trigeminal nerve.

### [Solution to Problem]

The disclosure of the present application relates to a device and an attachment for applying transcutaneous electrical nerve stimulation to the second branch and/or the third branch of the trigeminal nerve illustrated below.
(1) A device for applying transcutaneous electrical nerve stimulation to a second branch and/or a third branch of a trigeminal nerve, the device including:
   an eyeglass-type frame wearable on a head of a user;
   electrodes held on the eyeglass-type frame; and
   an electric circuit for applying electric pulses to the electrodes,
   wherein when the second branch of the right face is defined as a right face second branch, the second branch of the left face is defined as a left face second branch, the third branch of the right face is defined as a right face third branch, and the third branch of the left face is defined as a left face third branch,
   the electrodes each include at least one electrode unit selected from a group consisting of
      a first electrode unit including at least two electrodes for selectively, electrically stimulating the right face second branch,
      a second electrode unit including at least two electrodes for selectively, electrically stimulating the left face second branch,
      a third electrode unit including at least two electrodes for selectively, electrically stimulating the right face third branch, and
      a fourth electrode unit including at least two electrodes for selectively, electrically stimulating the left face third branch, and
   wherein the electric circuit is configured to form a circuit between the at least two electrodes of the first electrode unit, form a circuit between the at least two electrodes of the second electrode unit, form a circuit between the at least two electrodes of the third electrode unit, and form a circuit between the at least two electrodes of the fourth electrode unit.
(2) The device according to (1) above,
   wherein the eyeglass-type frame includes at least a right rim, a left rim, a right nose pad, a left nose pad, a right temple, and a left temple,
   wherein at least one of the electrodes forming the first electrode unit is arranged on a right nose pad portion, and/or at least one of the electrodes forming the second electrode unit is arranged on a left nose pad portion,
   wherein the third electrode unit is arranged on the right temple, and
   wherein the fourth electrode unit is arranged on the left temple.
(3) The device according to (1) above,
   wherein the eyeglass-type frame includes at least a right rim, a left rim, a right nose pad, a left nose pad, a right temple, and a left temple,
   wherein at least one of the electrodes forming the first electrode unit is arranged under the right rim, and/or at least one of the electrodes forming the second electrode unit is arranged under the left rim,
   wherein the third electrode unit is arranged on the right temple, and
   wherein the fourth electrode unit is arranged on the left temple.
(4) The device according to (1) above further including the first electrode unit, the second electrode unit, the third electrode unit, and the fourth electrode unit.
(5) The device according to (1) above, wherein at least one electrode unit selected from a group consisting of the first electrode unit, the second electrode unit, the third electrode unit, and the fourth electrode unit includes three or more electrodes.
(6) The device according to any one of (1) to (5) above further including a control unit,
   wherein the control unit controls a pulse parameter of electric pulses to be applied to at least one electrode unit selected from a group consisting of the first electrode unit, the second electrode unit, the third electrode unit, and the fourth electrode unit.
(7) The device according to (6) above further including a measuring unit,
   wherein the measuring unit measures at least one electrical resistance or at least one value of current selected from a group consisting of an electrical resistance across, or a value of current flowing between, the electrodes of the first electrode unit, an electrical resistance across, or a value of current flowing between, the electrodes of the second electrode unit, an electrical resistance across, or a value of current flowing between, the electrodes of the third electrode unit, and an electrical resistance across, or a value of current flowing between, the electrodes of the fourth electrode unit, and
   wherein the control unit controls the pulse parameter based on a measurement result from the measuring unit.
(8) The device according to (7) above, wherein when the measurement result from the measuring unit exceeds a preset range,
   the control unit
   stops application of electric pulses to an electrode unit where the preset range is exceeded, and/or
   issues a hazard signal recognizable by a user.
(9) The device according to (6) above further including a communication circuit for a user to control the control unit via wired and/or wireless connection from an external device other than the device.
(10) The device according to any one of (1) to (5) above further including a control switch operated manually by a user,
   wherein the control switch is configured to manually switch the pulse parameter of electric pulses to be applied to at least one electrode unit selected from a group consisting of the first electrode unit, the second electrode unit, the third electrode unit, and the fourth electrode unit.
(11) The device according to any one of (1) to (5) above further including an elastic member configured to push at least one of the electrodes included in the electrode unit in a direction away from the eyeglass-type frame.
(12) The device according to any one of (1) to (5) above further including a power supply unit for applying electric pulses to the electrodes.
(13) An attachment for applying transcutaneous electrical nerve stimulation to a second branch and/or a third branch of a trigeminal nerve, the attachment including:
   at least one attachment selected from a group consisting of
      a first attachment mountable on at least one selected from a group consisting of a bridge, a right nose pad, and a right rim of an eyeglasses,
      a second attachment mountable on at least one selected from a group consisting of the bridge, a left nose pad, and a left rim of the eyeglasses,
      a third attachment mountable on the right rim of the eyeglasses, and
      a fourth attachment mountable on the left rim of the eyeglasses,
   wherein when the second branch of the right face is defined as a right face second branch, the second branch of the left face is defined as a left face second branch, the third branch of the right face is defined as a right face third branch, and the third branch of the left face is defined as a left face third branch,
   the first attachment includes
      a first electrode unit including at least two electrodes for selectively, electrically stimulating the right face second branch, and
      an electric circuit configured to form a circuit between the at least two electrodes of the first electrode unit and to apply electric pulses,
   the second attachment includes
      a second electrode unit including at least two electrodes for selectively, electrically stimulating the left face second branch, and
      an electric circuit configured to form a circuit between the at least two electrodes of the second electrode unit and to apply electric pulses,
   the third attachment includes
      a third electrode unit including at least two electrodes for selectively, electrically stimulating the right face third branch, and
      an electric circuit configured to form a circuit between the at least two electrodes of the third electrode unit and to apply electric pulses. and
   the fourth attachment includes
      a fourth electrode unit including at least two electrodes for selectively, electrically stimulating the left face third branch, and
      an electric circuit configured to form a circuit between the at least two electrodes of the fourth electrode unit and to apply electric pulses.
(14) The attachment according to (13) above, wherein the first attachment and the second attachment are connected via a connection part.

### [Advantageous Effects of Invention]

The use of eyeglasses holding the device and the attachment for applying transcutaneous electrical nerve stimulation to the second branch and/or the third branch of the trigeminal nerve disclosed in the present application makes it possible to apply transcutaneous electrical nerve stimulation to the second branch and/or the third branch of the trigeminal nerve. Further, since the device and the attachment for applying transcutaneous electrical nerve stimulation to the second branch and/or the third branch of the trigeminal nerve disclosed in the present application are configured such that the electrode unit is held on an eyeglass-type frame, at least a part of the electrode unit is hidden by the eyeglass-type frame. Therefore, compared to the devices disclosed in Patent Literatures 1 and 2, the pressure of having to worry about the appearance from others is reduced. Further, since the device and the attachment for applying transcutaneous electrical nerve stimulation to the second branch and/or the third branch of the trigeminal nerve disclosed in the present application are configured such that the electrode unit is held on an eyeglass-type frame, it is possible for the user to easily position the stimulus electrode to a position suitable for transcutaneous electrical nerve stimulation on the second branch and/or the third branch of the trigeminal nerve without having to look in the mirror or having to paying particular attention.

### [Brief Description of Drawings]

[FIG. 1]

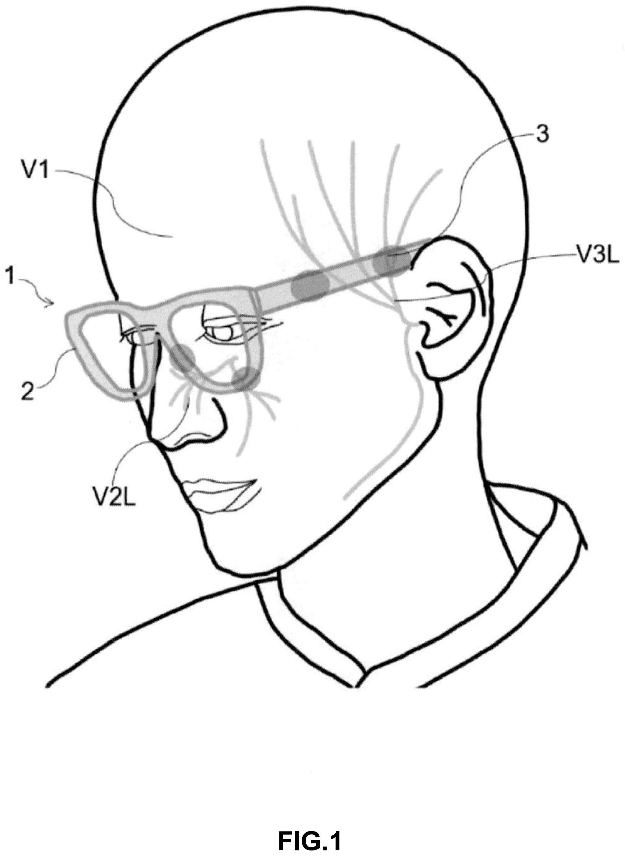

   FIG. 1 is a schematic diagram when the user is wearing the device.
[FIG. 2]

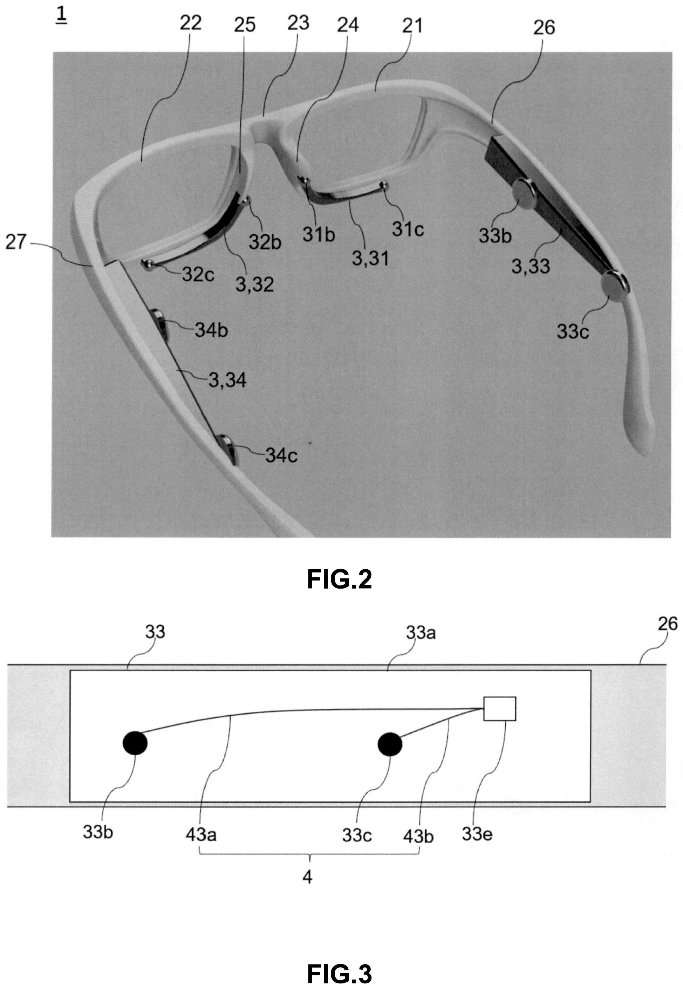

   FIG. 2 is a schematic diagram illustrating an example of the device.
[FIG. 3]
   FIG. 3 is a diagram illustrating an overview of the third electrode unit.
[FIG. 4A]

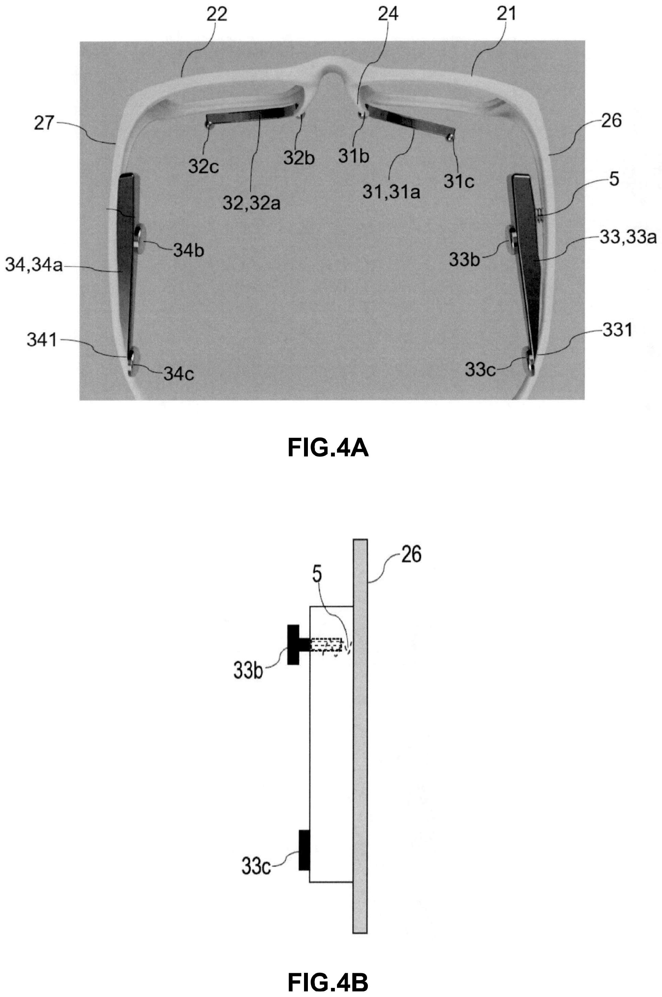

   FIG. 4A is a schematic diagram illustrating use example 1 of the elastic member.
[FIG. 4B]
   FIG. 4B is a schematic diagram illustrating use example 2 of the elastic member.
[FIG. 4C]

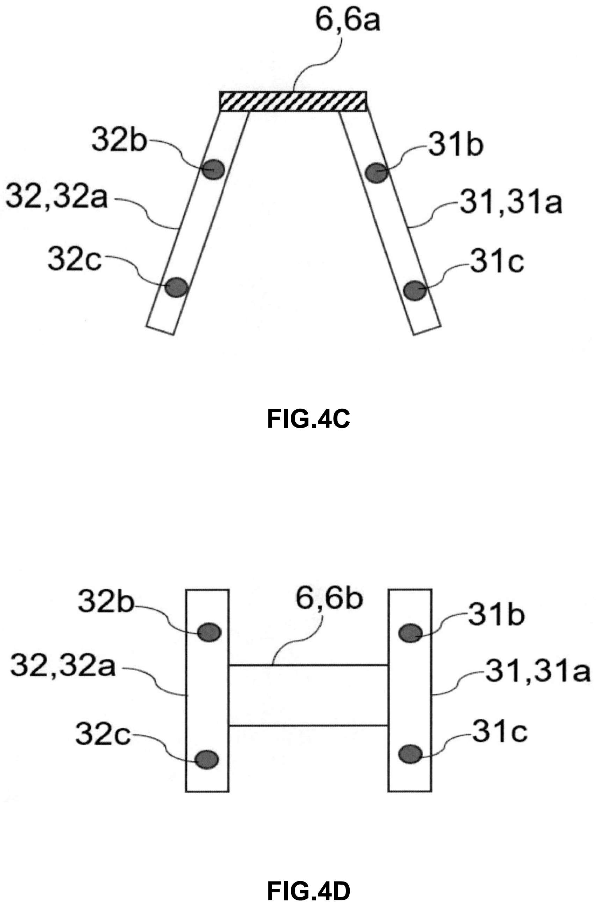

   FIG. 4C is a schematic diagram illustrating an arrangement example of the first electrode unit and the second electrode unit.
[FIG. 4D]
   FIG. 4D is a schematic diagram illustrating an arrangement example of the first electrode unit and the second electrode unit.
[FIG. 4E]

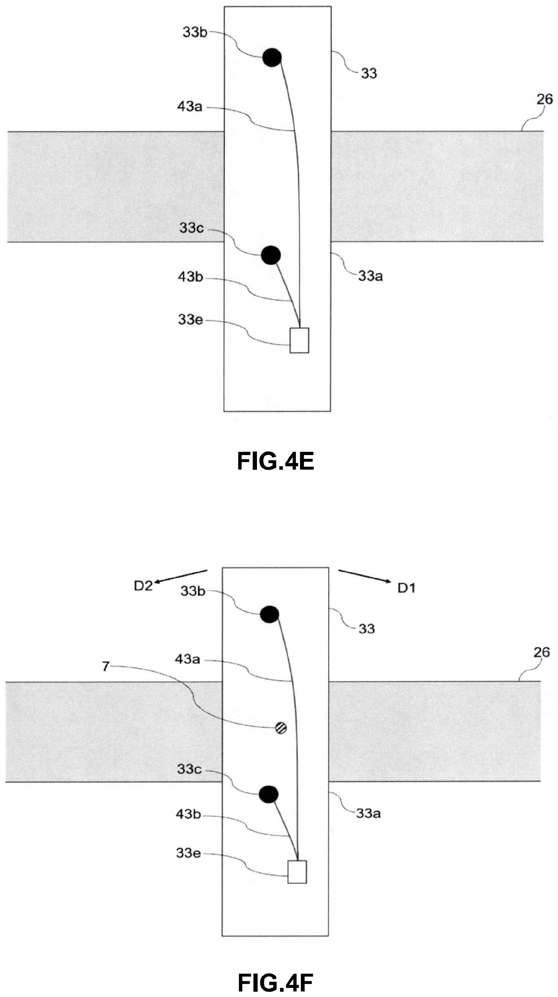

   FIG. 4E is a schematic diagram illustrating an arrangement example of the third electrode unit and the fourth electrode unit that stimulate the third branch.
[FIG. 4F]
   FIG. 4F is a schematic diagram illustrating an arrangement example of the third electrode unit and the fourth electrode unit that stimulate the third branch.
[FIG. 5]

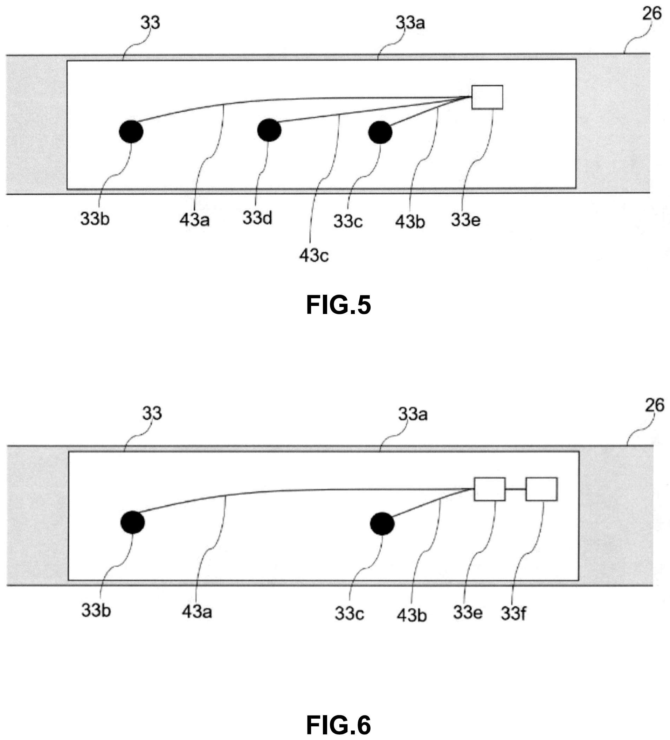

   FIG. 5 is a schematic diagram illustrating the number of electrodes included in each electrode unit.
[FIG. 6]
   FIG. 6 is a schematic diagram illustrating a control unit.
[FIG. 7]

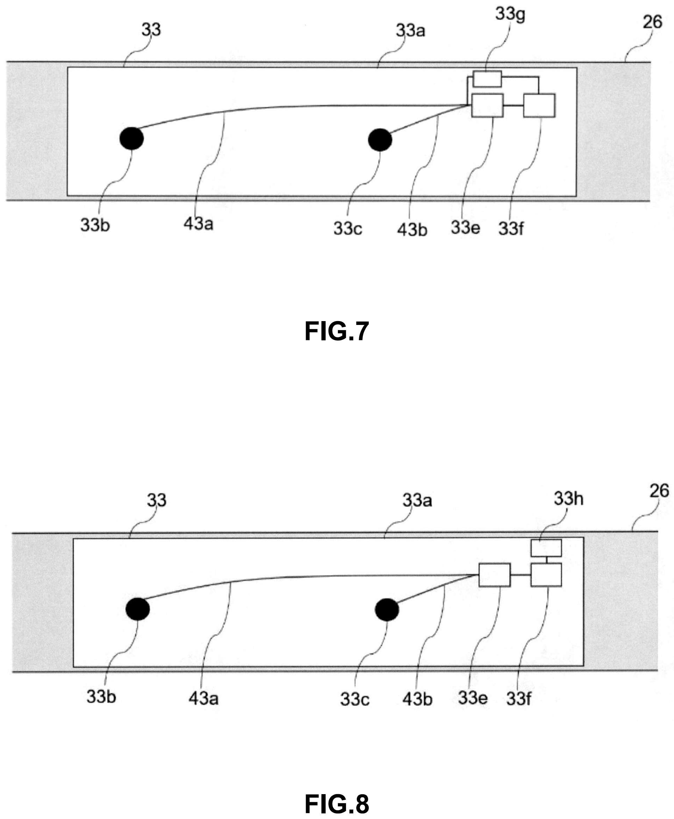

   FIG. 7 is a schematic diagram illustrating a measuring unit.
[FIG. 8]
   FIG. 8 is a schematic diagram illustrating a communication circuit.
[FIG. 9]

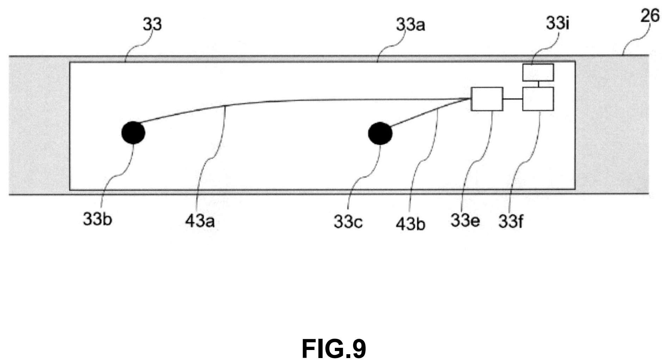

   FIG. 9 is a schematic diagram illustrating a control switch operated manually by the user.

### [Description of Embodiments]

A device for applying transcutaneous electrical nerve stimulation to the second branch and/or the third branch of the trigeminal nerve (hereafter, which may be simply referred to as "device") and an attachment for applying transcutaneous electrical nerve stimulation to the second branch and/or the third branch of the trigeminal nerve (hereafter, which may be simply referred to as "attachment") disclosed in the present application will be described below in detail. Note that the position, size, range, or the like of each configuration illustrated in the drawings do not always represent the actual position, size, range, or the like in order to facilitate the understanding. Thus, the disclosure of the present application is not necessarily limited to the position, size, range, or the like disclosed in the drawings.

Further, in the present specification,
(1) numerical ranges expressed by using "to" mean a range including numerical values stated before and after "to" as the lower limit value and the upper limit value, respectively;
(2) numerical values, numerical ranges, and qualitative expressions (for example, expressions such as "identical", "same", or the like) represent the numerical values, numerical ranges, and properties including errors generally accepted in this technical field; and
(3) when "substantially XX shape" is stated, a shape recognized as not only accurately XX shape but also approximately XX shape is included.

Further, when there are multiple members having the identical/same or similar function, these members are denoted with addition of ordinal numbers such as "first XX", "second XX", "third XX", or "fourth XX" or denoted with addition of alphabets such as "XXa", "XXb", "XXc", or "XXd", for example. When description common to respective members is provided, however, these members may be simply denoted as "XX" without the addition of ordinal numbers or alphabets.

### [Embodiment of the device]

First, the overview of a device 1 according to the embodiment will be described with reference to FIG. 1 to FIG. 3. FIG. 1 is a schematic diagram when the user is wearing the device. FIG. 2 is a schematic diagram illustrating an example of the device. FIG. 3 is a diagram illustrating an overview of a third electrode unit 33.

The device 1 according to the embodiment is used to apply transcutaneous electrical nerve stimulation to the second branch and/or the third branch of the trigeminal nerve. The second branch and the third branch will be described in detail with reference to FIG. 1. The trigeminal nerve is one of the peripheral nerves that transmit facial sensations to the brain. Trigeminal nerves are distributed all over the skin and serve as a sensor for information on sensations such as a sensation from light touch (tactile sense), a pain (pain sensation), a sensation of hot or cold (temperature sensation), or the like. The trigeminal nerve is divided into three from a trigeminal ganglion, specifically, a part from the forehead to the eyes is categorized as a first branch (ophthalmic nerve) territory, a part from the lower eyelids to the cheek, upper lip, and upper gum is categorized as a second branch (maxillary nerve) territory, and a part from the lower lip to the lower jaw, lower gum, half of the tongue, temporal region, frontotemporal region, temples, preauricular region, and tragus is categorized as a third branch (mandibular nerve) territory. Note that, in the example illustrated in FIG. 1, the second branch and the third branch of the left face of a human body are illustrated. In the present specification, the second branch of the left face is defined as a left face second branch V2L, and the third branch of the left face is defined as a left face third branch V3L. Further, although not illustrated in FIG. 1, the second branch and the third branch of the right face are present in the right face having V2L and V3L symmetrical about the face midline as the axis. In the present specification, the second branch of the right face is defined as a right face second branch V2R, and the third branch of the right face is defined as a right face third branch V3R. Further, when collectively described, the right face second branch V2R and the left face second branch V2L are simply denoted as the second branch, and when collectively described, the right face third branch V3R and the left face third branch V3L are simply denoted as the third branch. Further, when collectively described, the second branch and the third branch are simply denoted as the branch.

The device 1 according to the embodiment includes an eyeglass-type frame 2 that is wearable on the head of the user, electrodes 3 held by the eyeglass-type frame 2, and an electric circuit 4 for applying electric pulses to the electrodes.

As illustrated in FIG. 2, the eyeglass-type frame 2 includes at least a right rim 21 for holding a lens, a left rim 22 for holding a lens, a bridge 23 for connecting the right rim 21 and the left rim 22 to each other, a right nose pad 24, a left nose pad 25, a right temple 26, and a left temple 27.

The electrodes 3 include at least one electrode unit selected from a group consisting of:
- a first electrode unit 31 including at least two electrodes (31b, 31c) for selectively, electrically stimulating the right face second branch V2R;
- a second electrode unit 32 including at least two electrodes (32b, 32c) for selectively, electrically stimulating the left face second branch V2L;
- a third electrode unit 33 including at least two electrodes (33b, 33c) for selectively, electrically stimulating the right face third branch V3R; and
- a fourth electrode unit 34 including at least two electrodes (34b, 34c) for selectively, electrically stimulating the left face third branch V3L.

The first electrode unit 31, the second electrode unit 32, the third electrode unit 33, and the fourth electrode unit 34 can be arranged in any portions of the eyeglass-type frame 2 so as to be able to selectively, electrically stimulate the right face second branch V2R, the left face second branch V2L, the right face third branch V3R, and the left face third branch V3L, respectively. While the first electrode unit 31, the second electrode unit 32, the third electrode unit 33, and the fourth electrode unit 34 can be of any form as long as any one or more electrode units are arranged on the eyeglass-type frame 2, all the four electrode units may be of course arranged on the eyeglass-type frame 2. When any one or more electrode units are arranged on the eyeglass-type frame 2, any combination of the electrode units can be employed. The combination may be, but not limited to, for example, a combination of the first electrode unit 31 and the second electrode unit 32 for electrically stimulating the second branch, a combination of the third electrode unit 33 and the fourth electrode unit 34 for electrically stimulating the third branch, a combination of the first electrode unit 31 and the third electrode unit 33 for electrically stimulating the right face, a combination of the second electrode unit 32 and the fourth electrode unit 34 for electrically stimulating the left face, or the like.

The way of holding the first electrode unit 31, the second electrode unit 32, the third electrode unit 33, and the fourth electrode unit 34 on the eyeglass-type frame 2 is not particularly limited as long as respective electrode units are attached to the eyeglass-type frame 2 so as to be able to electrically stimulate respective branches. Although not limited, the electrode units and the eyeglass-type frame 2 may be sticked by an adhesive agent, the electrode units and the eyeglass-type frame 2 may be formed in shapes that can be fitted and thereby fitted to each other, the eyeglass-type frame 2 and the electrode units may be magnetically coupled by using magnets, or the like.

The electric circuit (not illustrated in FIG. 2) is configured to form a circuit between at least two electrodes (31b, 31c) of the first electrode unit 31, form a circuit between at least two electrodes (32b, 32c) of the second electrode unit 32, form a circuit between at least two electrodes (33b, 33c) of the third electrode unit 33, and form a circuit between at least two electrodes (34b, 34c) of the fourth electrode unit 34.

The electrodes 3 and the electric circuit 4 will be described in more detail with reference to FIG. 3. FIG. 3 is a schematic diagram of the third electrode unit 33 illustrated in FIG. 2 when viewed from the left temple 27 side. In the example illustrated in FIG. 3, the third electrode unit 33 has a third insulating substrate 33a in which a pair of a third electrode 33b and a third electrode 33c are formed. Further, the pair of the third electrode 33b and the third electrode 33c are connected to a third power supply unit 33e via a third wire 43a and a third wire 43b (hereafter, which may be simply referred to as "wire 43" when collectively described). Note that, in the example illustrated in FIG. 3, the wire 43 is formed on the surface of the third insulating substrate 33a. When covered with an insulating material, the wire 43 may be formed on the surface of the third insulating substrate 33a. Alternatively, the wire 43 may be arranged inside the third insulating substrate 33a. When the device 1 is worn by the user, the pair of the third electrode 33b and the third electrode 33c are in contact with the skin of the user. Thus, electric pulses applied from the third power supply unit 33e form an electric circuit in the order of the third wire 43a, the third electrode 33b, the skin, the third electrode 33c, the third wire 43b, and the third power supply unit 33e, for example, and thereby only the right face third branch V3R can be selectively, electrically stimulated.

The insulating substrate 33a is not particularly limited as long as the pair of the third electrode 33b and the third electrode 33c are not short-circuited. For example, an insulating material may be used. The insulating substrate 33a may be a flexible material whose shape is changeable at ordinary temperature or may be a rigid material whose shape is unchangeable at ordinary temperature.

The flexible material whose shape is changeable at ordinary temperature may be, but not limited to, for example, a rubber such as silicone rubber; a resin such as polypropylene, polyurethane elastomer, or polyvinyl chloride; sponge such as ethylene-propylene-diene monomer (EPDM) or ethylene propylene rubber (EPR); or the like. The flexible material whose shape is changeable at ordinary temperature may be solid or may be hollow. When the flexible material is hollow, the degree of a change in shape is larger, and this has an advantageous effect that the pair of the third electrode 33b and the third electrode 33c are easily fitted closely to the face when the device 1 is worn thereon.

The rigid material whose shape is unchangeable at ordinary temperature may be, but not limited to, for example, ceramics, glass, thermosetting resin, insulating coating-treated metal, or the like.

The pair of the third electrode 33b and the third electrode 33c are not particularly limited as long as they can apply electric pulses to the user when coming into contact with the skin of the face. The pair of the third electrode 33b and the third electrode 33c may be a solid type electrode made of metal or the like or may be a wet type pad-like electrode with hydrogel or the like used in health equipment or the like.

The third power supply unit 33e is not particularly limited as long as it can apply electric pulses to the pair of the third electrode 33b and the third electrode 33c and may be a battery or the like. Further, the third power supply unit 33e is not particularly limited in the arranged position as long as it can be connected to the wire 43 and may be arranged inside the third insulating substrate 33a or may be arranged at a position different from the third insulating substrate 33a. Note that, although the third power supply unit 33e is illustrated in the example illustrated in FIG. 3, the third power supply unit 33e can also be attached by the user before the use of the device 1. Therefore, the third power supply unit 33e is not an essential configuration but is an optional and additional configuration when the device 1 is provided. Therefore, the electric circuit 4 in the present specification means a circuit for electrically connecting the third power supply unit 33e and the third electrodes (33b, 33c) to each other when the third power supply unit 33e is arranged, and the third power supply unit 33e is not a requirement of the electric circuit.

Although not illustrated, the first electrode unit 31, the second electrode unit 32, and the fourth electrode unit 34 can be fabricated in the same manner as the third electrode unit 33 illustrated in FIG. 3 except that respective shapes of the insulating substrates are changed as needed in accordance with arranged locations. The configuration of the first electrode unit 31 can be defined by replacing "a/the third insulating substrate 33a", "a/the pair of a/the third electrode 33b and a/the third electrode 33c", "a/the third wire 43a and a/the third wire 43b", and "a/the third power supply unit 33e" of the third electrode unit 33 with "a/the first insulating substrate 31a", "a/the pair of a/the first electrode 31b and a/the first electrode 31c", "a/the first wire 41a and a/the first wire 41b", and "a/the first power supply unit 31e", respectively. The configuration of the second electrode unit 32 can be defined by replacing the same with "a/the second insulating substrate 32a", "a/the pair of a/the second electrode 32b and a/the second electrode 32c", "a/the second wire 42a and a/the second wire 42b", and "a/the second power supply unit 32e" in the same manner as the first electrode unit 31. The configuration of the fourth electrode unit 34 can be defined by replacing the same with "a/the fourth insulating substrate 34a", "a/the pair of a/the fourth electrode 34b and a/the fourth electrode 34c", "a/the fourth wire 44a and a/the fourth wire 44b", and "a/the fourth power supply unit 34e" in the same manner as the first electrode unit 31.

Note that the example in which the electrode units each have a power supply unit is described for the third electrode unit 33, which is illustrated in FIG. 3, and the first electrode unit 31, the second electrode unit 32, and the fourth electrode unit 34, which are defined through the replacement described above. Alternatively, in the device 1 according to the embodiment, when two or more electrode units selected from the first electrode unit 31, the second electrode unit 32, the third electrode unit 33, and the fourth electrode unit 34 are held on the eyeglass-type frame 2, the at least two or more electrode units may share a single power supply unit. For example, the four electrode units may share a single power supply unit, or the first electrode unit 31 and the second electrode unit 32 arranged at close positions may share a single power supply unit.

Further, when the eyeglass-type frame 2 is worn by the user, certain clearances occur in general between the user's face and the rims and between the user's face and the temples. Therefore, the thickness of the insulating substrate, the length of the electrode protruding out of the insulating substrate, and/or the like can be suitably adjusted so that the electrodes of respective electrode units are more likely to be in contact with the user's face in accordance with the location of each electrode unit arranged on the eyeglass-type frame 2.

The device 1 according to the embodiment, when worn by the user, can apply transcutaneous electrical nerve stimulation to at least one branch selected from a group consisting of the right face second branch V2R, the left face second branch V2L, the right face third branch V3R, and the left face third branch V3L. Therefore, the device according to the embodiment is expected to be used for treatment and/or alleviation of symptoms associated with neurological conditions and disorders, including migraine, tension headache, cluster headache, persistent unilateral headache, SUNCT, chronic paroxysmal unilateral headache, trigeminal neuralgia, facial nerve palsy, facial nerve disorders, connective tissue inflammation, chronic pain, depression, cyclothymia, post-traumatic stress disorder, post-concussion syndrome, fatigue, coma, anxiety, tremors, aphasia, obsessive-compulsive disorder, insomnia, sleep disorders, sleep apnea syndromes, hypersomnia, epilepsy, drop attacks, attention-deficit/hyperactivity disorder, Parkinson's disease, Alzheimer's disease, multiple sclerosis, stroke, and cerebellar syndromes; brain fog; post-COVID-19 condition; malaise; mood disorder; autonomic nervous system dysfunction; or the like. Further, the device according to the embodiment can be used as health equipment or the like.

The device 1 according to the embodiment achieves the following advantageous effects.
(1) At least one or more electrode units selected from a group consisting of the first electrode unit 31, the second electrode unit 32, the third electrode unit 33, and the fourth electrode unit 34 are held on the eyeglass-type frame 2 so as to be arranged between the eyeglass-type frame 2 and the user's face. Therefore, when the device 1 is worn by the user, each electrode unit is hidden by the eyeglass-type frame 2 and is less likely to be viewed from others. Thus, compared to such a type of devices that are attached to the forehead or the like, the pressure of having to worry about the appearance from others is reduced. Note that, with respect to the advantageous effects achieved when the device 1 disclosed in the present application is worn, it is not intended that all the electrode units are hidden when the device 1 is worn. As described above, as long as at least a part of the electrode unit is hidden by the eyeglass-type frame 2 and is thereby invisible from outside, the advantageous effect with the device 1 disclosed in the present application is achieved. It is of course preferable to adjust the design of the eyeglass-type frame 2 and the shape, the size, or the like of the electrode unit to increase the ratio of the invisible part of the electrode unit from outside.
(2) The devices disclosed in Patent Literatures 1 and 2 are required for the user by itself to attach electrodes to locations intended to electrically stimulate. Thus, there is a problem that putting on the device takes efforts, such as attaching the device while checking the attached position in front of a mirror. In contrast, the device 1 according to the embodiment is of the eyeglass type, and thus, once the user puts it on the head, the electrode units, by themselves, come into contact with the skin of the user at positions suitable for transcutaneous electrical nerve stimulation. Therefore, convenience in use of the device 1 is improved.
(3) The device 1 is of the eyeglass type, and this makes it possible to electrically stimulate the second branch and/or the third branch for a long time without involving an uncomfortable feeling even in front of others. Therefore, the device 1 can be used not only for treatment and/or alleviation of symptoms associated with neurological conditions and disorders described above but also for cosmetic purposes such as facelifts, a change of pace, or the like.
(4) Because the device 1 is of the eyeglass type, it is possible to enhance the design of the treatment device itself in terms of appearance, and this can enhance motivation for using the device 1 at patients who were reluctant to have treatment and/or alleviation of symptoms associated with neurological conditions and disorders described above. Further, because of being the eyeglass type, the user may easily put on or wear the device 1 at any place. As a result, for example, a patient suffering from migraine will use the device 1 earlier when feeling unwell, and this can reduce the usage of an oral medication during the acute phase.
(5) The device disclosed in Patent Literature 1 is attached to the face such that the pair of electrodes 11 and 12 extend across the face midline of the user. However, for example, even when the pair of electrodes of the device disclosed in Patent Literature 1 are attached to the location of the second branch across the face midline, electric current is merely conducted through the nasal cartilage, and it is not possible to electrically stimulate the second branch. In contrast, in the device 1 disclosed in the present application, each of the first electrode unit 31, the second electrode unit 32, the third electrode unit 33, and the fourth electrode unit 34 forms a single circuit, and electric pulses can be applied to the circuit. Therefore, transcutaneous electrical nerve stimulation can be applied to one or more desired locations selected from the right face second branch V2R, the left face second branch V2L, the right face third branch V3R, and the left face third branch V3L. Note that, when four electrode units are arranged, it is possible to apply transcutaneous electrical nerve stimulation to one or more desired locations by selecting one or more electrode units to which electric pulses are applied. It is therefore possible to selectively apply transcutaneous electrical nerve stimulation to one or more branches required for treatment or alleviation of symptoms.
(6) In Patent Literatures 1 and 2, the first branch is electrically stimulated. In contrast, the device disclosed in the present application can electrically stimulate the second branch and/or the third branch. The electrical stimulation to the second branch and/or the third branch can be used not only for treatment and/or alleviation of symptoms of the disease described above but also for modulation of pain sensations of the jaw or teeth. Therefore, the device 1 can be utilized for alleviation of pains during dental treatment.

### [Arrangement of electrode units]

As described above, the first electrode unit 31, the second electrode unit 32, the third electrode unit 33, and the fourth electrode unit 34 can be held on any portions of the eyeglass-type frame 2 so as to be able to selectively, electrically stimulate the right face second branch V2R, the left face second branch V2L, the right face third branch V3R, and the left face third branch V3L, respectively. However, various employable examples of the holding (arrangement) of the first electrode unit 31, the second electrode unit 32, the third electrode unit 33, and the fourth electrode unit 34 will be described.

### [Use example 1 of an elastic member]

The use example 1 of an elastic member will be described with reference to FIG. 4A. In the example illustrated in FIG. 4A, an elastic member 5 that pushes the third electrode unit 33 in a direction away from the right temple 26 (direction toward the face) is arranged between the third electrode unit 33 and the right temple 26. The elastic member 5 is not particularly limited as long as it can push the third electrode unit 33 in the direction described above and may be an elastic material such as a coil spring, a leaf spring, a rubber, or the like.

In the example illustrated in FIG. 4A, the elastic member 5 is arranged at a position of the third electrode unit 33 that is close to the right rim 21, an end 331 of the third electrode unit 33 that is distant from the right rim 21 is pivotably held on the right temple 26. In the example illustrated in FIG. 4A, the third electrode 33b is arranged at a position where a clearance is present between the right temple 26 and the face when the device 1 is worn on the head. However, the third electrode 33b is pushed in the face direction due to the arrangement of the elastic member 5, and this achieves an advantageous effect that the third electrode 33b and the face can be more reliably in contact with each other.

Note that the example illustrated in FIG. 4A is a mere example of the arrangement of the elastic member 5. As long as the technical concept that the elastic member 5 is arranged and thus the third electrode 33b and/or 33c of the third electrode unit 33 is pushed in the face direction can be realized, there is no restriction on the number and the arranged positions of elastic members 5. For example, instead of the pivotable holding of the end 331 illustrated in FIG. 4A, the elastic member 5 may be provided though not illustrated. In other words, the third electrode unit 33 may be held by the right temple 26 via two elastic members 5 without being in direct contact with the right temple 26. Alternatively, a single elastic member 5 may be arranged in a portion at or around the center of the third electrode unit 33, and thereby the third electrode unit 33 may be held by the right temple 26 via the single elastic member 5. That is, the whole or at least a part of the third electrode unit 33 may be held to be directly abutted on the right temple 26, or the third electrode unit 33 may be held by the right temple 26 indirectly via another member without being abutted on the right temple 26.

Although detailed description is omitted, the elastic member 5 may be arranged to any one or more of the portions between the fourth electrode unit 34 and the left temple 27, between the first electrode unit 31 and the right rim 21, and between the second electrode unit 32 and the left rim 22.

### [Use example 2 of the elastic member]

The use example 2 of the elastic member 5 will be described with reference to FIG. 4B. In the example illustrated in FIG. 4A, the elastic member 5 pushes the third insulating substrate 33a of the third electrode unit 33. In contrast, in the example illustrated in FIG. 4B, the elastic member 5 directly pushes the third electrode 33b, which differs from the example illustrated in FIG. 4A. Although an example in which the elastic member 5 is a coil spring is illustrated in FIG. 4B, the elastic member 5 is not particularly limited as long as it can push the third electrode 33b in the direction away from the right temple 26 (direction toward the face), such as a leaf spring, a rubber, or the like in the same manner as above.

Although an example in which the elastic member 5 is arranged so as to push the third electrode 33b is illustrated in FIG. 4B, the elastic member 5 may be arranged so as to push the third electrode 33b and/or the third electrode 33c. Although not illustrated, the elastic member 5 may be arranged so that any one or more electrodes of the fourth electrode 34b and/or 34c of the fourth electrode unit 34, the first electrode 31b and/or 31c of the first electrode unit 31, and the second electrodes 32b and/or 32c of the second electrode unit 32 can be pushed in the face direction.

When the examples illustrated in FIG. 4A and FIG. 4B are taken collectively, it can be said that the elastic member 5 is a member that pushes one or more electrodes of one or more electrode units directly or indirectly in the direction away from the eyeglass-type frame 2 (direction toward the face).

### [Arrangement example 1 of the first electrode unit 31 and the second electrode unit 32 that stimulate the second branch]

The arrangement example 1 of the first electrode unit 31 and the second electrode unit 32 will be described with reference to FIG. 2 and FIG. 4A. In the example illustrated in FIG. 2, the first electrode 31b, which is one of the electrodes forming the first electrode unit 31, is arranged on the right nose pad 24 of the eyeglass-type frame 2, and the first electrode 31c is arranged at a part under the right rim 21. Alternatively, although not illustrated, both of the first electrode 31b and the first electrode 31c may be arranged at portions of the right nose pad 24. Given the manner the second branch runs in the face, even when both of the first electrode 31b and the first electrode 31c are arranged at portions of the right nose pad 24, it is possible to electrically stimulate the right face second branch V2R.

Further alternatively, both of the first electrode 31b and the first electrode 31c may be arranged at a part under the right rim 21. Given the manner the second branch runs in the face, even when both of the first electrode 31b and the first electrode 31c are arranged at a part under the right rim 21, it is possible to electrically stimulate the right face second branch V2R. Suitable adjustment can be performed by adjusting the thickness of the first insulating substrate 31a, by using the elastic member 5 described above, or the like in accordance with the arranged locations of the first electrode 31b and the first electrode 31c so that the first electrode 31b and the first electrode 31c are more likely to be in contact with the user's face when the device 1 is worn.

Further, in the example illustrated in FIG. 4A, while the first electrode 31b of the first electrode unit 31 is arranged on (fixed to) the right nose pad 24, the first electrode 31c is spaced away from the right rim 21. That is, while a part of the first electrode unit 31 is fixed to the eyeglass-type frame 2, the remaining part of the first electrode unit 31 is separated from the eyeglass-type frame 2. In the case of the example illustrated in FIG. 4A, the first electrode unit 31 can be configured as follows.
(a) While the first electrode 31b (otherwise, the first insulating substrate 31a) is arranged on (fixed to) the right nose pad 24, the remaining part of the first insulating substrate 31a is configured pivotably about the first electrode 31b. In such a case, the position of the first electrode 31c can be adjusted.
(b) The first insulating substrate 31a is configured to have an extendable structure and/or to be deformable. The extendable structure may be, but not limited to, a telescopic structure or the like. Further, the deformable configuration may be, but not limited to, an articulated structure or a configuration with a material whose shape changes when subjected to a predetermined force (for example, an insulating coated metal or the like). When the first insulating substrate 31a is configured to have the extendable structure and/or to be deformable, the positional relationship between the first electrode 31b and the first electrode 31c can be adjusted in a wider range. The configuration described in (b) may be of course combined with the configuration described in (a).
(c) In the examples described above in (a) and (b), after the position of the first electrode 31c is adjusted, the first electrode 31c (otherwise, the first insulating substrate 31a holding the first electrode 31c) may be engaged with the underside of the right rim 21. In such a case, the stability of the first electrode unit 31 is improved. On the other hand, the first electrode 31c may be in a state separated from the right rim 21. In such a case, when the user is wearing the device 1, the first electrode 31c is more likely to be in contact with its face.
(d) The examples described above in (a) to (c) are examples in which the first electrode 31b is arranged on (fixed to) the right nose pad 24. Alternatively, the first electrode 31c may be arranged under (fixed to the underside of) the right rim 21, and the first electrode 31b may be separated from the eyeglass-type frame 2.

Although detailed description is omitted, the arrangement described above in (a) to (d) may be applied also for the second electrode 32b and the second electrode 32c of the second electrode unit 32. When the arrangement described above is taken collectively, it can be said that at least one of the electrodes (31b, 31c) forming the first electrode unit 31 is arranged on a portion of the right nose pad 24, and/or at least one of the electrodes (32b, 32c) forming the second electrode unit 32 is arranged on the portion of the left nose pad 25. Otherwise, it can be said that at least one of the electrodes (31b, 31c) forming the first electrode unit 31 is arranged under the right rim 21, and/or at least one of the electrodes (32b, 32c) forming the second electrode unit 32 is arranged under the left rim 22.

Further, although not illustrated, the technical concepts described above in (a) to (d) can be applied for the third electrode unit 33 and the right temple 26 as well as the fourth electrode unit 34 and the left temple 27. For example, the end 331 of the third electrode unit 33, which is close to the ear and is likely to be in contact with the face, may be arranged on (fixed to) the right temple, and the third insulating substrate 33a may be configured to have an extendable structure and/or to be deformable. The third electrode 33b side may be of course arranged on (fixed to) the right temple 26. Furthermore, the fourth electrode unit 34 may also be configured in the same manner as the third electrode unit 33.

### [Arrangement example 2 of the first electrode unit 31 and the second electrode unit 32 that stimulate the second branch]

The arrangement example 2 of the first electrode unit 31 and the second electrode unit 32 will be described with reference to FIG. 4C and FIG. 4D. In the arrangement example 1, the first electrode unit 31 and the second electrode unit 32 are configured as separate members. In contrast, in the arrangement example 2, the first electrode unit 31 and the second electrode unit 32 are connected via the connection part 6 and integrally handled, which makes a difference.

FIG. 4C illustrates an example in which the first electrode unit 31 and the second electrode unit 32 are connected via a bridge engagement member 6a as the connection part 6. Although not illustrated, the bridge engagement member 6a is configured to be engageable with the bridge 23 of the eyeglass-type frame 2. Therefore, in the example illustrated in FIG. 4C, when the bridge engagement member 6a is engaged with the bridge 23, the first insulating substrate 31a is configured to have an extendable structure and/or to be deformable as needed so that the first electrodes (31b, 31c) and the second electrodes (32b, 32c) are arranged, for example, at the positions in the arrangement example 1 described above, and thereby the positions of the first electrodes (31b, 31c) can be adjusted. The same adjustment can also be applied for the second electrode unit 32.

FIG. 4D illustrates an example in which the first electrode unit 31 and the second electrode unit 32 are connected via a nasal adhesive tape 6b as the connection part6. The nasal adhesive tape 6b is desirably formed of a material that can maintain a predetermined shape as with a nasal dilator tape, for example. When the nasal adhesive tape 6b is adhered to the nose, the first insulating substrate 31a is configured to have an extendable structure and/or to be deformable as needed so that the first electrodes (31b, 31c) and the second electrodes (32b, 32c) are arranged, for example, at the positions in the arrangement example 1 described above, and thereby the positions of the first electrodes (31b, 31c) can be adjusted. The same adjustment can be applied for the second electrode unit 32. Note that, when the nasal adhesive tape 6b, the first electrode unit 31, and the second electrode unit 32 are configured to be able to hold a predetermined shape, the nasal adhesive tape 6b is first locked to the nose, the eyeglass-type frame 2 is then put on, and thereby the first electrode unit 31 and the second electrode unit 32 can also be used to be pushed against the face by the eyeglass-type frame 2. In such a case, it can be said that the first electrode unit 31 and the second electrode unit 32 are held indirectly on the eyeglass-type frame 2. Naturally, for example, at least a part of the nasal adhesive tape 6b, the first electrode unit 31, and the second electrode unit 32 may be directly held on the eyeglass-type frame 2 by using magnets or the like.

### [Arrangement example of the third electrode unit 33 and the fourth electrode unit 34 that stimulate the third branch]

The arrangement example of the third electrode unit 33 and the fourth electrode unit 34 that stimulate the third branch will be described with reference to FIG. 4E and FIG. 4F. FIG. 2 and other figures illustrate an example in which the third electrode unit 33 is arranged along the right temple 26, and the fourth electrode unit 34 is arranged along the left temple 27. Alternatively or additionally, as illustrated in FIG. 4E, the third electrode unit 33 may be unparallel to the right temple 26, in other words, at least one of the third electrodes (33b, 33c) may be arranged at a position offset from the right temple 26.

Further, in the example illustrated in FIG. 4E, the third electrode unit 33 is arranged not pivotably about the right temple 26. Alternatively, as illustrated in FIG. 4F, the third electrode unit 33 may be arranged so as to be rotatable about the rotation axis 7 with respect to the right temple 26 (in the directions D1, D2), and the rotation can be locked at a desired position. When the third electrode unit 33 is arranged rotatably, this achieves an advantageous effect that the range where the right face third branch V3R can be stimulated is expanded, which makes it easier to find a location where transcutaneous electrical nerve stimulation is available. Although not illustrated, the fourth electrode unit 34 may also be configured in the same manner as the third electrode unit 33.

Next, an optional and additional configuration example that can be employed by the device 1 will be described.

### [The number of electrodes included in each electrode unit]

The number of electrodes included in each electrode unit will be described with reference to FIG. 5. The number of electrodes arranged in each electrode unit is not particularly limited as long as it is two or greater, which enables a pair of electrodes to be formed, and the number may be three as illustrated in FIG. 5. Further, although not illustrated, the number of electrodes included in an electrode unit may be four, five, six, seven, eight, nine, ten, or eleven or greater. When three or more electrodes are formed, respective electrodes can be connected to the power supply unit via wires. Note that the numbers of electrodes included in the first electrode unit 31, the second electrode unit 32, the third electrode unit 33, and the fourth electrode unit 34 may be the same or may differ from each other.

When three or more electrodes are formed, positions of the formed electrodes may be formed in a substantially straight shape at equal intervals as illustrated in FIG. 5. Alternatively, the electrodes may be formed at unequal intervals and/or in a non-straight shape. Note that, for the electrodes included in the first electrode unit 31 and the second electrode unit 32 for applying transcutaneous electrical nerve stimulation to the second branch, three or more electrodes can be arranged in the same manner as in the embodiment described above in "Arrangement of electrode units". More specifically, all the third electrodes forming the third electrode unit 33 may be arranged at portions of the right nose pad 24 or arranged under the right rim 21. Otherwise, at least one of the third electrodes may be arranged at a portion of the right nose pad 24, or at least one of the third electrodes may be arranged under the right rim 21. The fourth electrodes of the fourth electrode unit 34 can also be arranged in the same manner as the third electrodes.

The advantageous effects achieved when three or more electrodes are formed will be described with reference to the example illustrated in FIG. 5. Note that, while FIG. 5 illustrates the example of the third electrode unit 33, similar advantageous effects are achieved also for the first electrode unit 31, the second electrode unit 32, and the fourth electrode unit 34.
(1) In a case of two electrodes (33b and 33c), the location to be electrically stimulated is always the same. Thus, a long time of continued electrical stimulation may cause the user to feel discomfort such as a pain. In contrast, in the example illustrated in FIG. 5, for example, after two electrodes (33b and 33d) are used to apply transcutaneous electrical nerve stimulation to the right face third branch V3R for a predetermined time, different two electrodes (33d and 33c) can be used next to apply transcutaneous electrical nerve stimulation to the right face third branch V3R at a different location. Therefore, uncomfortable feeling at the user can be reduced when the user uses the device 1 for a long time.
(2) The effects may differ for users in accordance with the location of the right face third branch V3R subjected to transcutaneous electrical nerve stimulation. By selecting any two electrodes out of a plurality of arranged electrodes and applying electric pulses thereto, it is possible to widen a selection range of the location to which transcutaneous electrical nerve stimulation is applied, which increases the likelihood that the effect can be obtained.
(3) For example, when the electrode 33d is used as the positive pole and the electrodes 33b and 33c are used as the negative pole, the electrode 33d and the electrode 33b can form one circuit, and the electrode 33d and the electrode 33c can form another circuit. In such a case, when a measuring unit described later is provided, the resistance of the circuit A formed of the electrode 33d and the electrode 33b and the resistance of the circuit B formed of the electrode 33d and the electrode 33c can be measured separately. For example, even when the same voltage is applied to the circuit A and the circuit B, desired transcutaneous electrical nerve stimulation may be unable to be applied to the user due to an obstacle such as hairs or unsuitable contact between the electrode and the skin. However, by measuring the resistances of the circuit A and the circuit B separately, for example, it is possible to select the desirable one of the circuits to apply electrical stimulation.

### [Control unit]

The control unit will be described with reference to FIG. 6. The device 1 may include a control unit 33f. In the example illustrated in FIG. 6, the control unit 33f is arranged in the third electrode unit 33. In the example illustrated in FIG. 6, the control unit 33f controls pulse parameters of electric pulses to be applied to the pair of the electrode 33b and the electrode 33c of the third electrode unit 33.

The pulse parameters controlled by the control unit 33f are not particularly limited as long as they can be used for treatment and/or alleviation of symptoms associated with neurological conditions and disorders described above as examples. The pulse parameters may be, but not limited to, for example, a pulse frequency, a duration of a single pulse applied, an interval time between applied pulses, a value of conducted current, a pulse shape, or the like. The control unit 33f can control one or more selected from the pulse parameters illustrated as examples.

The range of each pulse parameter will be described below though not limited thereto.
- Pulse frequency: 1 Hz to 10 MHz, preferably, 60 Hz to 200 Hz.
- Duration of a single pulse applied (pulse on-time): 10 nsec to 1000 µsec, preferably, 250 µsec to 300 µsec.
- Interval time between applied pulses: 90 nsec to 999 msec, preferably, 4.7 msec to 16.4 msec.
- Value of current: 1 mA to 40 mA, preferably, 6 mA to 16 mA.
- Pulse shape: biphasic wave, preferably, biphasic square wave.
- Value of voltage: a voltage that allows current having the value of current described above to flow.

Although not illustrated, the first electrode unit 31, the second electrode unit 32, and the fourth electrode unit 34 may include the same control unit as the control unit 33f, respectively. Alternatively, the device 1 may include one to three control units, and two or more electrode units selected from the first electrode unit 31, the second electrode unit 32, the third electrode unit 33, and the fourth electrode unit 34 may share a single control unit. The pulse parameters may be controlled separately for each of the first electrode unit 31, the second electrode unit 32, the third electrode unit 33, and the fourth electrode unit 34, or the pulse parameters may be controlled so that electric pulses having the same pulse parameters can be applied to at least two or more electrode units.

When the device 1 includes the control unit to control the pulse parameters, the following advantageous effects are achieved.
(1) Electric pulses can be applied using pulse parameters, such as the amount of current, for example, having the maximum range acceptable to the user. Therefore, the effect of treatment and/or the effect of alleviation of symptoms can be enhanced.
(2) For some thickness of the subcutaneous fat layer, it is possible to adjust the frequency to adjust the depth reached by the current.

### [Measuring unit]

The measuring unit will be described with reference to FIG. 7. The device 1 may include the measuring unit. In the example illustrated in FIG. 7, the measuring unit 33g measures the electrical resistance or the value of current between the electrode 33b and the electrode 33c of the third electrode unit 33.

In the device 1 disclosed in the present application, each electrode unit is held on the eyeglass-type frame 2. When the user puts the eyeglass-type frame 2 on the head, the wearing condition may change every time the user puts it on, for example, the eyeglass-type frame 2 may be tilted, the user wears the eyeglass-type frame 2 with hairs, a mask strap, or the like being present on the ear under the eyeglass-type frame 2, or the like. Further, the eyeglass-type frame 2 may be hit by a hand or the like while worn by the user and thereby may be tilted. In such a case, the degree of contact between an electrode of each electrode unit and the skin of the user may change, in other words, the area of the electrode in contact with the skin may change. Even when the first electrode unit 31 and the second electrode unit 32 are indirectly held on the eyeglass-type frame 2, the area of the electrode in contact with the skin may change. Accordingly, a change in the area of the electrode in contact with the skin may cause a change in the value of current flowing through the skin, an increased value of flowing current may damage a muscle or a nerve, and a reduced value of flowing current may reduce the effect obtained by wearing the device 1.

Because the measuring unit 33g is provided, the electrical resistance across, or the value of current flowing between the pair of the electrode 33b and the electrode 33c can be always measured. The measuring unit 33g is not particularly limited as long as it can measure the electrical resistance or the value of current between the pair of the electrode 33b and the electrode 33c, and a known measuring device (measuring circuit) for electrical resistances or values of current can be used. When the electrical resistance is measured, the value of current can be calculated from the value of the applied voltage. The control unit 33f controls the pulse parameter (for example, the applied voltage or the like) based on the measurement result from the measuring unit 33g, and it is thereby possible to perform control such as adjustment of the value of current flowing between the pair of the electrode 33b and the electrode 33c, for example. Further, when the applied voltage is reduced, the control unit 33f may control the pulse parameter such as the frequency and/or the pulse width in order to obtain the same effect of the electrical stimulation. Further, when the measurement result from the measuring unit 33g exceeds a preset range, for example, when the measurement value exceeds the range and thereby excessive current flows or when the measurement value falls below the range and thereby the effect of the device 1 is no longer obtained, the control unit 33f may stop the application of electric pulses to the third electrode unit 33 and/or may issue a hazard signal that is recognizable by the user. The hazard signal is not particularly limited as long as it enables the user to recognize occurrence of an anomaly, such as an alert sound, signal display, or the like.

Although not illustrated, the first electrode unit 31, the second electrode unit 32, and the fourth electrode unit 34 may include the same measuring unit as the measuring unit 33g, respectively. Alternatively, the device 1 may include one to three measuring units, and two or more electrode units selected from the first electrode unit 31, the second electrode unit 32, the third electrode unit 33, and the fourth electrode unit 34 may share a single measuring unit. Note that the preset range may be the same or may differ for respective electrode units.

When the device 1 includes the measuring unit, the following advantageous effects are achieved.
(1) The devices of Patent Literature 1 and Patent Literature 2 are of the type that requires the electrodes to be attached to the user. Thus, the contact area of the electrode in contact with the skin of the user is less likely to change. In contrast, since the device 1 disclosed in the present application holds the electrode units on the eyeglass-type frame 2, the area of the electrode in contact with the skin of the user may change. Because the measuring unit is provided and application of electric pulses is stopped when an anomaly is detected, the safety in use of the device 1 disclosed in the present application is enhanced.
(2) Since the measurement result from the measuring unit is fed back to the control unit and thereby stable electric pulses can be applied, the effect obtained by using the device 1 can be further improved.
(3) It is also possible to detect and control an electrode unit that is optimal for electrical stimulation based not only on the measured value of electrical resistance or value of current but also on information on usability or the like provided from the user who has worn the device 1. Further, also when three or more electrodes are arranged at a single electrode unit and thereby two or more circuits are formed, it is possible to detect and control an optimal circuit.

### [Communication circuit]

The communication circuit will be described with reference to FIG. 8. The device 1 may include the communication circuit 33h. In the example illustrated in FIG. 8, the use of communication circuit 33h enables the user to control the control unit 33f via wired and/or wireless connection from an external device other than the device 1.

When connected to an external device via wired connection, the communication circuit 33h can include a connector for the connection to the external device and wires for the connection between the connector and the control unit 33f. When connected to an external device via wireless connection, a receiver of a known wireless unit such as Bluetooth can be embedded into the circuit. The external device may be a smartphone or the like. Note that, when a rechargeable battery or the like are used as the third power supply unit 33e, the communication circuit may be used as a charging circuit for the third power supply unit 33e.

Although not illustrated, the first electrode unit 31, the second electrode unit 32, and the fourth electrode unit 34 may include the same communication circuit as the communication circuit 33h, respectively. Alternatively, the device 1 may include one to three communication circuits, and two or more electrode units selected from the first electrode unit 31, the second electrode unit 32, the third electrode unit 33, and the fourth electrode unit 34 may share a single communication circuit.

When the device 1 includes the communication circuit, the following advantageous effects are achieved.
(1) It is possible to change the control detail of the control unit of the device 1 via an application in a smartphone or the like or provide a control instruction for the pulse parameter or the like to the control unit directly from the smartphone or the like. Furthermore, it is also possible to charge the third power supply unit 33e while using the device 1. Further, it is also possible to accumulate the usage status or the effect in an application and perform machine learning or the like to provide an instruction for an electrical stimulation pattern optimized for the user. Therefore, the convenience in use of the device 1 is improved.
(2) Because the communication circuit is provided, when an anomaly occurs in the device 1, an anomaly occurrence signal can be transmitted not only to the user but also to a manager or the like. Therefore, the safety is enhanced even when the device 1 is used by a person having a disorder in an organ that perceives a hazard signal.

### [Manually operated control switch]

A control switch operated manually by the user (hereafter, which may be simply referred to as "switch") will be described with reference to FIG. 9. The device 1 may include the switch 33i. In the example illustrated in FIG. 9, the switch 33i is for manually performing control of the pulse parameters described above. The switch 33i is not particularly limited as long as it can control the pulse parameters described above. The switch may be, but not limited to, a switch for simply switching ON/OFF, a switch whose controlled output is changed by a long press or multiple operations, a capacitive switch, or the like. For example, it is possible to manually change the applied voltage or the like.

Although not illustrated, the first electrode unit 31, the second electrode unit 32, and the fourth electrode unit 34 may include the same switch as the switch 33i, respectively. Alternatively, the device 1 may include one to three switches, and two or more electrode units selected from the first electrode unit 31, the second electrode unit 32, the third electrode unit 33, and the fourth electrode unit 34 may share a single switch. Note that the output controlled by the switch may be the same or may differ for respective electrode units.

When the device 1 includes the switch, the following advantageous effect is achieved.
(1) It is possible to control the pulse parameters or the like by using only the configuration of the device 1 even without using a communication device such as a smartphone.

### [Lens]

Lenses that achieve a synergistic effect with the treatment and/or alleviation of neurological symptoms provided by the device 1 may be attached to the right rim 21 and the left rim 22. For example, for patients suffering from migraine, it is known that headaches are exacerbated by photic stimulation with a large amount of light, blue light, or red or yellow wavelengths, or flickering photic stimulation, and contrarily, headaches are alleviated by photic stimulation with green light at wavelengths of around 525 nm. Therefore, when a blue light blocking lens, a polarized lens, or a photochromatic lens is mounted on each of the right rim 21 and the left rim 22 of the device 1, a synergistic effect of the trigeminal nerve electrical stimulation and the headache alleviation made by intervention to the optic nerve can be expected. Typical vision correction lenses may be of course mounted on the right rim 21 and the left rim 22.

Note that the embodiment, the arrangement of the electrode units, and optional and additional configuration examples described above are more specific examples of the device 1 and are not limited thereto. Various design changes can be made as long as they fall in the scope of the technical concept disclosed in the present application. Further, any forms selected from the embodiment, the arrangement of the electrode units, and optional and additional configuration examples described above may be combined with each other.

### [Embodiment of the attachment]

Next, the attachment for applying transcutaneous electrical nerve stimulation to the second branch and/or the third branch of the trigeminal nerve will be described. When the attachment is mounted on existing eyeglasses, the existing eyeglasses can be used as the device 1 for applying transcutaneous electrical nerve stimulation to the second branch and/or the third branch of the trigeminal nerve.

The attachment according to the embodiment includes at least one attachment selected from a group consisting of:
- a first attachment that can be mounted on at least one selected from a group consisting of the bridge 23, the right nose pad 24, and the right rim 21 of eyeglasses,
- a second attachment that can be mounted on at least one selected from a group consisting of the bridge 23, the left nose pad 25, and the left rim 22 of the eyeglasses,
- a third attachment that can be mounted on the right rim 21 of the eyeglasses, and
- a fourth attachment that can be mounted on the left rim of the eyeglasses.

Further, when the second branch of the right face is defined as the right face second branch V2R, the second branch of the left face is defined as the left face second branch V2L, the third branch of the right face is defined as the right face third branch V3R, and the third branch of the left face is defined as the left face third branch V3L, each attachment includes the following configurations.

The first attachment includes:
- the first electrode unit 31 including at least two electrodes (31b, 31c) for selectively, electrically stimulating the right face second branch V2R, and
- the electric circuit configured to form a circuit between the at least two electrodes (31b, 31c) of the first electrode unit 31 to apply electric pulses.

The second attachment includes:
- the second electrode unit 32 including at least two electrodes (32b, 32c) for selectively, electrically stimulating the left face second branch V2L, and
- the electric circuit configured to form a circuit between the at least two electrodes (32b, 32c) of the second electrode unit 32 to apply electric pulses.

The third attachment includes:
- the third electrode unit 33 including at least two electrodes (33b, 33c) for selectively, electrically stimulating the right face third branch V3R, and
- the electric circuit configured to form a circuit between the at least two electrodes (33b, 33c) of the third electrode unit 33 to apply electric pulses.

The fourth attachment includes:
- the fourth electrode unit 34 including at least two electrodes (34b, 34c) for selectively, electrically stimulating the left face third branch V3L, and
- the electric circuit configured to form a circuit between the at least two electrodes (34b, 34c) of the fourth electrode unit 34 to apply electric pulses.

The first electrode unit 31, the second electrode unit 32, the third electrode unit 33, and the fourth electrode unit 34 of the attachment according to the embodiment are the same as the first electrode unit 31, the second electrode unit 32, the third electrode unit 33, and the fourth electrode unit 34 of the device 1 described above, respectively. Further, the electric circuits of the attachments according to the embodiment each are the same as the electric circuit 4 of the device 1 described above. Accordingly, to avoid duplicated description, the detailed description thereof is omitted.

Further, in at least one of the first to fourth attachments, optionally and additionally, the arrangement of electrodes in each attachment may be adjusted in the same manner as in "Arrangement of electrode units" described above in the embodiment of the device 1. Further, optionally and additionally, at least one of the attachments may employ the configurations described above in "The number of electrodes included in each electrode unit", "Control unit", "Measuring unit", "Communication circuit", and "Manually operated control switch". Further, a power supply unit may be included. In other words, the features described in the embodiment of the device 1 can also be employed in the first to fourth attachments. That is, the attachment according to the embodiment can employ the same configuration as the device 1 according to the embodiment except that the eyeglass-type frame 2 is excluded from the device 1 according to the embodiment. Therefore, the first attachment and the second attachment may be provided in a state of being connected via the connection part. Further, when any one or more attachments out of the first to fourth attachments are provided, any combination of the attachments is available.

### [Industrial Applicability]

The device and attachment disclosed in the present application enables the user to have transcutaneous electrical nerve stimulation applied to the second branch and/or the third branch of the trigeminal nerve without having to worry about the appearance from others. Therefore, the device and attachment disclosed in the present application are useful in the medical industry.

### [List of Reference Symbols]

- 1: device
- 2: eyeglass-type frame
- 21: right rim
- 22: left rim
- 23: bridge
- 24: right nose pad
- 25: left nose pad
- 26: right temple
- 27: left temple
- 3: electrode
- 31: first electrode unit
- 31a: first insulating substrate
- 31b, 31c: first electrode
- 32: second electrode unit
- 32a: second insulating substrate
- 32b, 32c: second electrode
- 33: third electrode unit
- 33a: third insulating substrate
- 33b, 33c, 33d: third electrode
- 33e: third power supply unit
- 33f: control unit
- 33g: measuring unit
- 33h: communication circuit
- 33i: switch
- 331: end
- 34: fourth electrode unit
- 34a: fourth insulating substrate
- 34b, 34c: fourth electrode
- 341: end
- 4: electric circuit
- 43a: third wire
- 43b: third wire
- 5: elastic member
- 6: connection part
- 6a: bridge engagement member
- 6b: nasal adhesive tape
- 7: rotation axis
- V2R: right face second branch
- V2L: left face second branch
- V3R: right face third branch
- V3L: left face third branch

## Claims

1. A device for applying transcutaneous electrical nerve stimulation to a second branch and/or a third branch of a trigeminal nerve, the device comprising:
an eyeglass-type frame wearable on a head of a user;
electrodes held on the eyeglass-type frame; and
an electric circuit for applying electric pulses to the electrodes,
wherein when the second branch of the right face is defined as a right face second branch, the second branch of the left face is defined as a left face second branch, the third branch of the right face is defined as a right face third branch, and the third branch of the left face is defined as a left face third branch,
the electrodes include at least one electrode unit selected from a group consisting of
a first electrode unit including at least two electrodes for selectively, electrically stimulating the right face second branch,
a second electrode unit including at least two electrodes for selectively, electrically stimulating the left face second branch,
a third electrode unit including at least two electrodes for selectively, electrically stimulating the right face third branch, and
a fourth electrode unit including at least two electrodes for selectively, electrically stimulating the left face third branch, and
wherein the electric circuit is configured to form a circuit between the at least two electrodes of the first electrode unit, form a circuit between the at least two electrodes of the second electrode unit, form a circuit between the at least two electrodes of the third electrode unit, and form a circuit between the at least two electrodes of the fourth electrode unit.

2. The device according to claim 1,
wherein the eyeglass-type frame includes at least a right rim, a left rim, a right nose pad, a left nose pad, a right temple, and a left temple,
wherein at least one of the electrodes forming the first electrode unit is arranged on a right nose pad portion, and/or at least one of the electrodes forming the second electrode unit is arranged on a left nose pad portion,
wherein the third electrode unit is arranged on the right temple, and
wherein the fourth electrode unit is arranged on the left temple.

3. The device according to claim 1,
wherein the eyeglass-type frame includes at least a right rim, a left rim, a right nose pad, a left nose pad, a right temple, and a left temple,
wherein at least one of the electrodes forming the first electrode unit is arranged under the right rim, and/or at least one of the electrodes forming the second electrode unit is arranged under the left rim,
wherein the third electrode unit is arranged on the right temple, and
wherein the fourth electrode unit is arranged on the left temple.

4. The device according to claim 1 further comprising the first electrode unit, the second electrode unit, the third electrode unit, and the fourth electrode unit.

5. The device according to claim 1, wherein at least one electrode unit selected from a group consisting of the first electrode unit, the second electrode unit, the third electrode unit, and the fourth electrode unit includes three or more electrodes.

6. The device according to any one of claims 1 to 5 further comprising a control unit,
wherein the control unit controls a pulse parameter of electric pulses to be applied to at least one electrode unit selected from a group consisting of the first electrode unit, the second electrode unit, the third electrode unit, and the fourth electrode unit.

7. The device according to claim 6 further comprising a measuring unit,
wherein the measuring unit measures at least one electrical resistance or at least one value of current selected from a group consisting of an electrical resistance across, or a value of current flowing between, the electrodes of the first electrode unit, an electrical resistance across, or a value of current flowing between, the electrodes of the second electrode unit, an electrical resistance across, or a value of current flowing between, the electrodes of the third electrode unit, and an electrical resistance across, or a value of current flowing between, the electrodes of the fourth electrode unit, and
wherein the control unit controls the pulse parameter based on a measurement result from the measuring unit.

8. The device according to claim 7, wherein when the measurement result from the measuring unit exceeds a preset range,
the control unit
stops application of electric pulses to an electrode unit where the preset range is exceeded, and/or
issues a hazard signal recognizable by a user.

9. The device according to claim 6 further comprising a communication circuit for a user to control the control unit via wired and/or wireless connection from an external device other than the device.

10. The device according to any one of claims 1 to 5 further comprising a control switch operated manually by a user,
wherein the control switch is configured to manually switch the pulse parameter of electric pulses to be applied to at least one electrode unit selected from a group consisting of the first electrode unit, the second electrode unit, the third electrode unit, and the fourth electrode unit.

11. The device according to any one of claims 1 to 5 further comprising an elastic member configured to push at least one of the electrodes included in the electrode unit in a direction away from the eyeglass-type frame.

12. The device according to any one of claims 1 to 5 further comprising a power supply unit for applying electric pulses to the electrodes.

13. An attachment for applying transcutaneous electrical nerve stimulation to a second branch and/or a third branch of a trigeminal nerve, the attachment comprising:
at least one attachment selected from a group consisting of
a first attachment mountable on at least one selected from a group consisting of a bridge, a right nose pad, and a right rim of an eyeglasses,
a second attachment mountable on at least one selected from a group consisting of the bridge, a left nose pad, and a left rim of the eyeglasses,
a third attachment mountable on the right rim of the eyeglasses, and
a fourth attachment mountable on the left rim of the eyeglasses,
wherein when the second branch of the right face is defined as a right face second branch, the second branch of the left face is defined as a left face second branch, the third branch of the right face is defined as a right face third branch, and the third branch of the left face is defined as a left face third branch,
the first attachment includes
a first electrode unit including at least two electrodes for selectively, electrically stimulating the right face second branch, and
an electric circuit configured to form a circuit between the at least two electrodes of the first electrode unit and to apply electric pulses,
the second attachment includes
a second electrode unit including at least two electrodes for selectively, electrically stimulating the left face second branch, and
an electric circuit configured to form a circuit between the at least two electrodes of the second electrode unit and to apply electric pulses,
the third attachment includes
a third electrode unit including at least two electrodes for selectively, electrically stimulating the right face third branch, and
an electric circuit configured to form a circuit between the at least two electrodes of the third electrode unit and to apply electric pulses. and
the fourth attachment includes
a fourth electrode unit including at least two electrodes for selectively, electrically stimulating the left face third branch, and
an electric circuit configured to form a circuit between the at least two electrodes of the fourth electrode unit and to apply electric pulses.

14. The attachment according to claim 13, wherein the first attachment and the second attachment are connected via a connection part.
